# EUROPÄISCHE PATENTSCHRIFT

(11) **EP 0 386 308 B1**
(45) Veröffentlichungstag und Bekanntmachung des Hinweises auf die Patenterteilung: **07.02.1996**
(21) Anmeldenummer: 89113809.1
(22) Anmeldetag: 26.07.1989
(51) Int. Cl.: A61B 17/60

(54) **Einseitiger verlängerbarer Aussenspanner zur Behandlung von Knochenerkrankungen**
Unilateral extensible external fixator for the treatment of bone injuries
Fixateur externe monolatéral allongeable pour le traitement des maladies des os

(30) Priorität: 06.03.1989 DE 8902691 U
(43) Veröffentlichungstag der Anmeldung: 12.09.1990
(73) Patentinhaber: Pfeil, Joachim Dr., D-69118 Heidelberg (DE); Veith, Wolfgang Dr., D-69124 Heidelberg (DE)
(72) Erfinder: Pfeil, Joachim Dr., D-69118 Heidelberg (DE); Veith, Wolfgang Dr., D-69124 Heidelberg (DE)
(74) Vertreter: Weiss, Ursula, Dr.

(56) Entgegenhaltungen:
- EP-A- 0 024 256
- EP-A- 0 153 546
- FR-A- 2 557 933
- GB-A- 2 110 094
- US-A- 4 615 338

## Beschreibung

Die Erfindung betrifft einen einseitigen verlängerbaren Außenspanner zur Behandlung von Knochenerkrankungen, bei dem aus einem rohrförmigen Zentralteil an beiden Enden mindestens je ein Kolben unabhängig von dem anderen Kolben ausfahrbar angeordnet ist, an dessen äußerem Ende verschiedene Verbindungsteile drehbar befestigbar sind.

Aus der EP-A-0 024 256 ist ein derartiger Außenspanner bekannt. An dem einen Ende dieses bekannten Außenspanners ist ein Kolben angeordnet, der durch Drehen einer Verstellmutter definiert verstellbar ist. An dem anderen Ende dieses bekannten Außenspanners befindet sich ein Kolben, mit dem eine teleskopartige Verstellung möglich ist. Für den Operateur besteht bei der Verwendung dieses bekannten Außenspanners der Nachteil, daß an jedem Ende in Anpassung an die Erfordernisse des Patienten lediglich eine der Verstellmöglichkeiten gegeben ist. Bei unterschiedlichen Behandlungserfordernissen, wie Sie im Folgenden beschrieben sind, ist es je nach Fall notwendig, jedes Ende unabhängig von dem anderen Ende sowohl definiert als auch teleskopartig verstellen zu können.

Unilaterale Schraubenfixateure sind zur Behandlung von Knochenerkrankungen der Extremitäten bereits bekannt. Die Vielzahl der unterschiedlichen Knochenerkrankungen stellen unterschiedliche Anforderungen für die äußerliche Fixation. Vorteilhaft ist deshalb ein Baukastensystem, das die unterschiedlichen Anforderungen erfüllen kann. Das beschriebene Gerät erfüllt vielfältige Aufgaben der äußeren Fixation. Mit diesem Gerät ist die Frakturversorgung auch von Zweietagenbrüchen möglich, desweiteren die Verlängerung mono- und bifokal, sowie motorgesteuerte Verlängerungen und Kallusstimulation. Im Rahmen der Pseudarthrosenbehandlung besteht die Möglichkeit der Fragmentverschiebung. Desweiteren kann mit dem beschriebenen Gerät eine kontinuierliche Umstellung in allen Bewegungsrichtungen durchgeführt werden.

Ein weiterer einseitiger Außenspanner ist aus dem Dokument EP-A-0 153 546 bekannt. Bei diesem Fixateur sind zwei Halter durch ein zwischen ihnen angeordnetes Verbindungsglied derart miteinander verbunden, daß die beiden Halter gegeneinander sowohl durch Drehen einer Verstellmutter definiert axial verstellbar als auch teleskopisch axial verstellbar sind, wobei bei beiden Verstallarten die beiden Halter gegen ein Verdrehen zueinander gesichert werden können.

Vergleichbare orthopädische Vorrichtungen, wie beispielsweise die in der DE-PS 3 543 042 beschriebenen (Orthofix), können nur Teile der vorgenannten Indikationen abdecken, insbesondere sind bislang Versorgungen von Zweietagenbrüchen, bifokale Verlängerungen und kontinuierliche Umstellungen sowie motorgesteuerte Verlängerungen und Fragmentverschiebungen mit dem Monofixateur nicht möglich. Bei diesen Indikationen waren bislang operationstechnisch schwieriger anzubringende Fixateure notwendig, die von mehreren Richtungen her die Haut perforieren. Nachteilig war bei den bisherig verfügbaren orthopädischen Vorrichtungen auch die Notwendigkeit je nach Indikationsstellung ein vollständig neues Gerät zu verwenden, da zum Beispiel nicht mit einem Gerät sowohl die Frakturversorgung als auch die Verlängerung durchgeführt werden konnte. Nachteilig an bisherigen Systemen war auch die Notwendigkeit,verschiedene Teile während der Behandlung zusätzlich auf den Fixateur aufzubringen, was oftmals dazu führte, daß Teile des Fixateurs während der Behandlung verlorengingen, was unnötige Mehrkosten verursachte.

Die der vorliegenden Erfindung zugrunde liegende Aufgabe besteht somit darin, einen einseitigen verlängerbaren Außenspanner zur Behandlung von Knochenerkrankungen vorzuschlagen, wobei die betroffenen Knochensegmente über Knochenschrauben, die in Klemmbacken gehalten werden, beliebig einstellbar sind, gegeneinander fixiert, verspannt, definiert verschoben und verdrehsicher geführt dynamisiert werden können.

Diese Aufgabe wird erfindungsgemäß dadurch gelöst, daß jeder Kolben wahlweise sowohl durch Drehen einer Verstellmutter definiert verstellbar ist als auch teleskopartig verstellbar ist, wobei sowohl bei der definierten als auch bei der teleskopartigen Verstellung die Kolben und das Zentralteil gegen ein Verdrehen gesichert sind.

Im Rahmen der Erfindung ist es sowohl vorgesehen, nur zwei Kolben in dem Zentralteil als auch innerhalb dieser Kolben zwei weitere Kolben ebenfalls teleskopartig ausfahrbar anzuordnen. Die Verbindungsteile werden jeweils an dem innersten Kolben befestigt.

Die hier vorgestellte orthopädische Vorrichtung erlaubt es, durch die spezielle Ausfahrbarkeit des doppelten Teleskopes sowie durch die hohe Stabilität auch der gelenkigen Verbindungen gegenüber Biegebelastungen mit nur einem Fixateurtyp für die untere Extremität sowie mit einem weiteren Fixateurtyp für die obere Extremität auszukommen. Dies bedeutet, daß in der Vorratshaltung einer orthopädischen oder unfallchirurgischen Klinik weniger verschiedene Fixateurtypen vorhanden sein müssen, zum anderen ist das Indikationsfeld dieser orthopädischen Vorrichtung weitaus größer als bei vergleichbaren Monofixateuren. Die orthopädische Vorrichtung ist so bautechnisch konzipiert, daß vor Behandlungsbeginn mit entsprechender Fragestellung nach dem Baukastenprinzip verschiedene Teile aufgebracht werden. Während der Behandlung wird dann kein Teil entfernt, so daß auch nicht die Gefahr des Verlierens einzelner Teile des Fixateurs besteht.

Die erzielbaren Vorteile lassen sich wie folgt zusammenfassen:

Die hier beschriebene orthopädische Vorrichtung erreicht mit je einer Dimensionierung für die untere und für die obere Extremität die Behandlungsmöglichkeit für alle Knochenerkrankungen, bei denen die Indikation zu einem Fixateur besteht. Während der Behandlung werden keine Teile auf die orthopädische Vorrichtung an- oder abgeschraubt, so daß nicht die Gefahr des Verlierens einzelner Teile während der Behandlung besteht. Die Handhabung ist technisch verständlich und einfach. Trotzdem kann ein großes Indikationsfeld durch diese orthopädische Vorrichtung abgedeckt werden.

Besonders bevorzugte Ausführungsformen des erfindungsgemäßen einseitigen verlängerbaren Außenspanners sind in den Ansprüchen beschrieben.

Mittels der ausfahrbaren Kolben kann eine bestimmte Länge betreffend des Zentralteils und der beiden Kolben eingestellt werden. An den Kolben können verschiedene Verbindungsteile mittels eines Klemmstiftes befestigt werden, woran wiederum Knochenschraubenklemmbacken anschraubbar sind. Als Verbindungsteile sind gemäß besonders bevorzugten Ausführungsformen verschiedene Teile vorgesehen, beispielsweise eine gelenkige Verbindung, die mittels Klemmstift auf das Kolbenende aufsteckbar ist und die beliebige räumliche Einstellung und Fixierung einer aufgeschraubten Knochenschraubenklemmbacke gestattet.

Diese Knochenschraubenklemmbacken können in Längsrichtung und/oder in Querrichtung (als sogenannte T-Backe) angeschraubt werden.

Ferner ist vorteilhafterweise als Verbindungsteil ein starres Zwischenteil vorgesehen, das ebenfalls mittels Klemmstift auf das Kolbenende aufsteckbar ist und diesem gegenüberliegend ein Innengewinde zum Anschrauben einer Knochenschraubenklemmbacke besitzt.

Ebenfalls ist als Verbindungsteil ein sogenannter Umsteller vorgesehen, der über einen selbsthemmenden Schneckentrieb die definierte Schwenkbewegung der daran angebrachten Plattform erreicht.

Ferner ist es möglich, als Verbindungsteil ein an beliebiger Stelle klemmbares Scharniergelenk vorzusehen, das bei Lösung der Klemmung zur Dynamisierung von Gelenken in der Ebene der Scharnierkippbewegung dient.

Eine rhythmische Aus- und Einwärtsbewegung des Kolbens bewirkt über die Schanzschrauben eine schwellende Mikrobewegung der Frakturstelle und somit eine Kallusstimulation, wobei verschiedene Ausführungsmöglichkeiten diesbezüglich möglich sind. Beispielsweise kann ein angeflanschter Schrittmotor so angesteuert werden, daß die Verstellmutter im gewünschten Rhythmus im und entgegen dem Uhrzeigersinn gedreht wird. Hierdurch würde sich der Kolben ebenfalls entsprechend ein- und auswärts bewegen und der Frakturstelle würde somit eine schwellende Mikrobewegung aufgeprägt werden.

Die drehbaren Verstellmuttern bewirken, wie beschrieben, die Einstellung der Kolben betreffend des Zentralteils.

Es ist eine vom Zentralteil lösbare Schraube vorgesehen, die auf dem Kolben verbleibt, um die Schnellverstellung zur Anpassung an die anatomischen Verhältnisse bzw. zum Zwecke der Dynamisierung zu ermöglichen.

Die Erfindung wird nun anhand der beigefügten Zeichnungen näher erläutert.

Es zeigen:
Figur 1 den Zentralteil und die beiden Kolben des Aussenspanners, teilweise geschnitten dargestellt,
Figur 2 die Ansicht der verschiedenen Teile des Außenspanners, teilweise geschnitten dargestellt,
Figur 3 die in Figur 2 dargestellten Teile in Seitenansicht, teilweise geschnitten dargestellt,
Figur 4 ein starres Zwischenteil,
Figur 5 ein klemmbares Scharniergelenk,
Figur 6 den Schnitt durch die Verstellmutter 8 und den Ring 12 gemäß Figur 8,
Figur 7 den Querschnitt der in Figur 6 dargestellten Verstellmutter 8 und des Ringes 12,
Figur 8 die teilweise geschnittene Ansicht eines weiteren Zentralteiles,
Figur 9 die Draufsicht auf das in Figur 1 dargestellte Zentralteil mit eingefahrenem Kolben 3 und
Figur 10 den Schnitt I-I gemäß Figur 9.

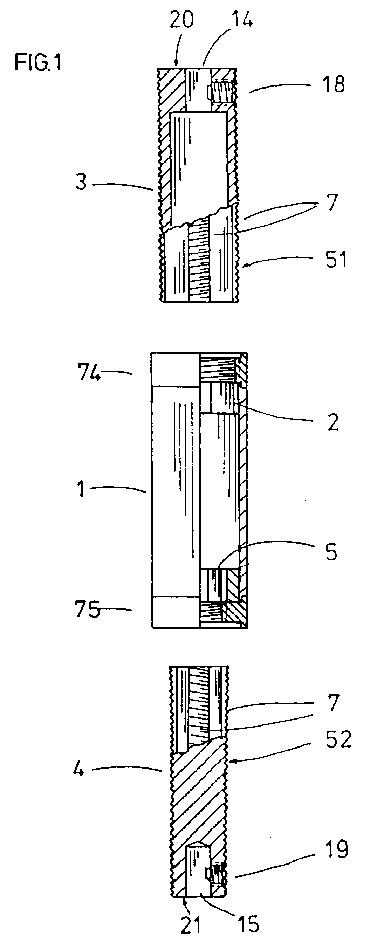

In Figur 1 sind im wesentlichen das Zentralteil 1 und die Kolben 3 und 4 des Außenspanners dargestellt. Das rohrförmige Zentralteil 1 ist an der Innenwand bereichsweise mit einem Innenkeilprofil 2 versehen, worin der hohlgebohrte Kolben 3 dadurch geführt wird, daß dieser an seinem Umfang über seine gesamte Längserstreckung die passende Außenkeilverzahnung 7 besitzt. Das Zentralteil 1 und der ausfahrbare Kolben 3 sind somit einerseits gegen ein Verdrehen gesichert, andererseits ist die freie axiale Bewegung der beiden Teile gewährleistet. Dies ist insbesondere bei der Dynamisierung von Bedeutung. Zur Verdeutlichung und Erklärung der einzelnen Teile sind in Figur 1 die einzelnen Teile voneinander entfernt gezeichnet, es ist jedoch aus der Beschreibung klar, daß so wohl der Kolben 3 als auch der Kolben 4 mit dem Zentralteil 1 zusammenwirken.

Der dem Kolben 3 gegenüberliegende Vollkolben 4 ist auch über seine gesamte Länge mit der Außenkeilverzahnung 7 versehen. Zu seiner Führung dient eine entsprechend innenverzahnte Muffe 5, die in das rohrförmige Ende des Zentralteils 1 eingepaßt ist.

Beide Kolben 3 und 4 sind nahezu vollständig im Zentralteil 1 bzw. ineinander (4 in 3) versenkbar. Hierdurch entspricht die minimale Längserstreckung des Außenspanners der Länge des Zentralteils 1. Die maximal erreichbare Länge wird durch beidseitiges Aus fahren der Kolben 3 und 4 erreicht, wobei das Zentralteil 1 um fast den Faktor drei verlängert wird. Vermindernd wirkt dabei die notwendige Überdeckung des Keilprofils an beiden Zentralteilenden, welche zur Sicherung der axialen Führung und der Kraftübertragung erforderlich ist.

Um eine definierte Verlängerung oder Verkürzung des Aussenspanners auch unter Krafteinwirkung zu ermöglichen, sind beide Kolben 3 bzw. 4 an den erhabenen Außenteilen - über die gesamte Länge - mit Gewindegängen 51 bzw. 52 vorzugsweise mit gleicher Steigung versehen. Die mit dem entsprechenden Innengewinde ausgestattete Verstellmutter 74 bzw. 75 erlaubt eine axiale Verstellung des Kolbens 3 bzw. 4 gegenüber dem Zentralteil 1. Die Verstellbarkeit ist hierbei für beide Kolben 3 bzw. 4 unabhängig voneinander.

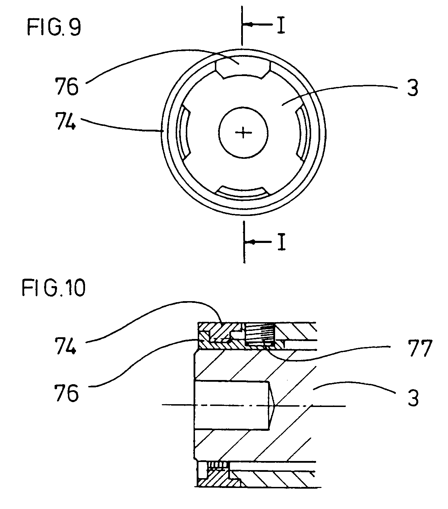

Die beiden Verstellmuttern 74 und 75 sind bis auf die gemäß den unterschiedlichen Kolbenquerschnitten ausgebildeten Innengewinde identisch. Die Wirkungsweise dieser Verstellmuttern 74 bzw. 75 wird anhand von Figur 9 und Figur 10 näher erläutert. Sie sind über ein oder mehrere Kulissensteine geführt.

Die Verstellmutter 74 bzw. 75 ist mit dem Zentralteil 1 drehbar verbunden. Durch das Drehen der Verstellmutter 74 bzw. 75 fährt der betreffende Kolben 3 bzw. 4 sinngemäß aus oder ein.

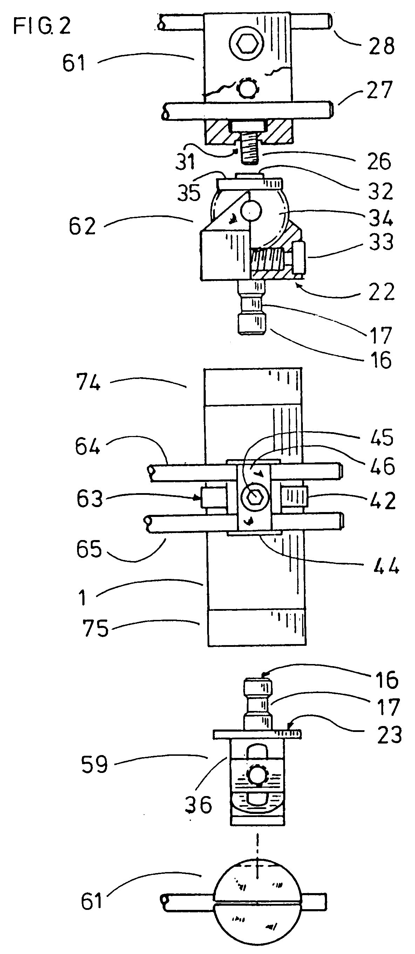

In Figur 2 sind verschiedene Teile des Außenspanners im auseinandergebauten Zustand teilweise geschnitten dargestellt.

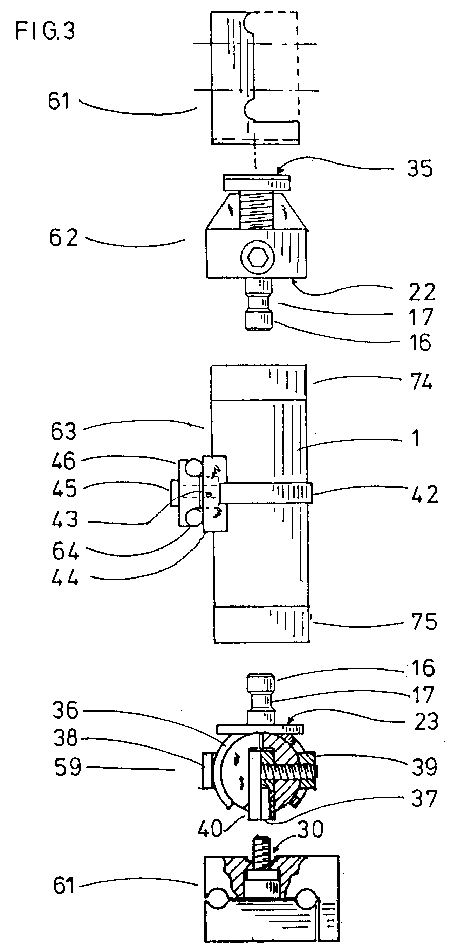

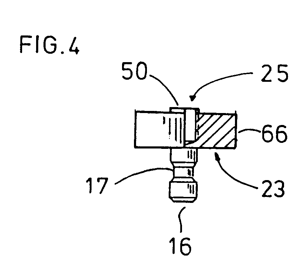

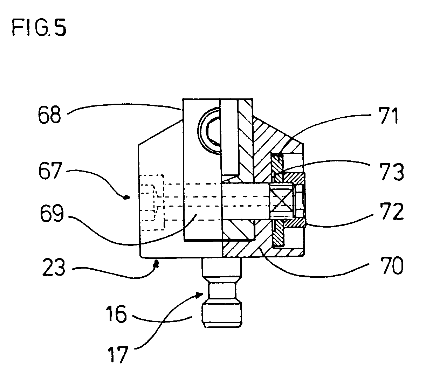

Figur 3 zeigt die in Figur 2 dargestellten Teile in Seitenansicht. Figur 2 und 3 werden im folgenden erläutert. Figur 4 und Figur 5 zeigen weitere mögliche Verbindungsteile des Außenspanners.

An dem äußeren Ende des Kolbens 3 bzw. 4 befindet sich gemäß Figur 1 die Bohrung 14 bzw. 15, in welche die in Figur 2, Figur 3, Figur 4 und 5 dargestellten beliebigen Verbindungsteile mittels eines Stiftes 16 angebaut werden können.

Der Stift 16 besitzt eine Ringnut 17 mit angeschrägten Flanken, worin eine an ihrem Ende entsprechend angeschrägte Madenschraube 18 bzw. 19 eingreifen kann (Figur 1). Sobald die Schraube 18 bzw. 19 leicht in die Ringnut 17 des in die Bohrung 14 bzw. 15 eingeführten Stiftes 16 eingreift, wird ein Herausgleiten desselben verhindert, wobei die freie Rotation jedoch erhalten bleibt. An der Stirnfläche des Kolbens 3 bzw. 4 befindet sich eine radiale Riffelung 20 bzw. 21 ähnlich der einer Hirth-Verzahnung. Eine entsprechende Riffelung 22 bzw. 23 befindet sich an der Unterseite der Verbindungsteile. Wird die Madenschraube 18 bzw. 19 ganz hineingedreht, so bewirken die Schrägen an Schrauben und Ringnut vollends ein Beiziehen des Zusatzteiles (Verbindungsteiles) gegen die Stirn des Kolbens, womit dann die radiale Verzahnung ein Verdrehen der beiden Teile gegeneinander verhindert.

Das oberste in Figur 2 und Figur 3 dargestellte Teil ist eine Knochenschraubenklemmbacke 61, die mittels einer in Achsrichtung liegenden Innensechskantschraube 26 an den unterhalb angeordneten Verbindungsteilen bzw. dem in Figur 4 und Figur 5 dargestellten Verbindungsteil befestigt werden kann. Zur Sicherung gegen ein Verdrehen der Backe 61 gegenüber dem jeweiligen Zusatzteil befindet sich an der Unterseite - im Bereich der Schraube - die Längsnut 31, in die ein entsprechender Längskeil 32 (bzw. 40, 50 oder 68) an der kommunizierenden Seite des Verbindungsteils eingreift. Die Klemmbacke 61 ist oberhalb des Schraubenkopfes in Längsrichtung mittig geteilt. Sie besitzt an den beiden Teilflächen halbrunde Nuten, die quer zur Mittelachse der Backe 61 verlaufen und der Aufnahme der Knochenschrauben 27 und 28 dienen. Deren Klemmung erfolgt durch Aufschrauben des Oberteils.

Der unterste Teil der Figur 2 und Figur 3 zeigt die beschriebene Klemmbacke 61 um 90 Grad gedreht, so daß die Verbindungsebene der beiden Knochenschrauben senkrecht zur Außenspannermittelachse steht. Hierbei erfolgt die Verschraubung der Klemmbacke 61 mit dem jeweiligen Verbindungsteil durch eine im Backenunterteil befindliche Innensechskantschraube. Auch hier befindet sich an der Klemmstelle eine Nut 30 zur Sicherung gegen ein Verdrehen.

Unterhalb der in Figur 2 und Figur 3 als oberstes Teil dargestellten Klemmbacke 61 befindet sich als Verbindungsteil der Umsteller 62. Mit diesem Teil wird über einen selbsthemmenden Schneckentrieb, der aus Schneckenwelle 33 und Schneckenrad 34 besteht, die definierte Schwenkbewegung der Plattform 35 erreicht. Auf diese Plattform 35 kann beispielsweise die oberhalb dargestellte Klemmbacke 61 verdrehsicher aufgeschraubt werden. Die Schnecke besitzt an ihrem Wellenende zur Betätigung einen Innensechskant.

Im unteren Bereich der Figur 2 ist als zweites Teil von unten ein gelenkiges Verbindungsteil 59 dargestellt, welches prinzipiell einen fixierbaren Kardan darstellt, der in Verbindung mit der Rotationsmöglichkeit des Klemmstiftes 16, der ebenfalls klemmbar ist, eine Schwenkbewegung der anschraubbaren Klemmbacken 61 erlaubt. Ein bereichsweise geschlitztes Kreissegment 36 umfaßt das zylinderförmige Innenteil 37. Die im Schlitz des Kreissegmentes 36 geführte Durchgangsschraube 38 durchdringt das Innenteil 37, welches damit um einen bestimmten Winkelbereich um seine Rotationsachse drehbar ist. Die Klemmung des Innenteils 37 erfolgt über eine Innensechskantschraube, wobei die Mutter 39 infolge einer der konvexen Außenseite des Kreissegments 36 angepaßten Form und einen in die Schlitzführung ragenden Keil nicht gegen ihre Auflage verdrehbar ist. Dies erlaubt ein Anziehen der Klemmschraube 38 mit nur einem Schlüssel ohne Gegenhalterung.

In einer schlitzförmigen Aussparung des Innenteils 37 befindet sich ein Vierkant 40, der in einer zur Zylinderachse senkrecht stehenden Ebene nach beiden Seiten schwenkbar ist und ebenfalls mittels der Durchgangsschraube 38 klemmbar ist. Zur Verbesserung der Rotationsstabilität ist im Bereich der Schraubenbohrung 38 eine radiale Riffelung, sowohl an beiden Seiten des Vierkants 40 als auch an den dem Vierkant zugewandten Innenseiten der Zylinderaussparung 37 vorgesehen. Der Vierkant 40 besitzt ein Innengewinde, worin mittels einer Schraube die Klemmbacke 61 anschraubbar ist.

Der mittlere Teil des in Figur 2 und Figur 3 dargestellten Außenspanners zeigt den Knochensegmentfixateur 63. Ein dem Außendurchmesser des Zentralteils 1 angepaßter Ring 42 wird nach Abnahme der Verstellmutter auf das Zentralteil 1 an beliebiger Stelle aufgebracht. Am Kreisringumfang befindet sich ein zylinderförmiges Teil 43 mit Innengewinde. Eine dem Umfang des Zentralteils 1 entsprechend geformte Plattform 44 umfaßt das Teil 43 und ist mit einer Aussparung im Bereich des unter ihr verlaufenden Kreisringes 42 versehen. Wird nun mittels der Schraube 45 über die obere Klemmbacke 46 und die dazwischen plazierten Knochenschrauben 64 und 65 auf die Plattform 44 Druck ausgeübt, so klemmt diese an der gewünschten Stelle auf dem Zentralteil 1 fest. Die Klemmbacke 46 ist hierbei in einem bestimmten Winkelbereich um die Achse der Schraube 45 drehbar, was die Ausrichtung der das Knochensegment haltenden Knochenschrauben 64 und 65 erlaubt. Um ein unerwünschtes Verdrehen zu verhindern, befindet sich an der Unterseite der Klemmbacke 46 und seiner Auflage 43 eine radiale Verzahnung, die bei der Klemmung ineinandergreift.

Figur 4 zeigt ein weiteres Verbindungsteil, das anstelle des in Figur 2 und Figur 3 dargestellten Zwischenteiles 62 oder 59 verwendet werden kann. Das in Figur 4 dargestellte starre Zwischenteil 66 weist als Klemmzapfen den eben beschriebenen Stift 16 auf. Ihm gegenüber befindet sich das Innengewinde 25 und der Keil 50, mit dem die beschriebene Knochenschraubenklemmbacke 61 verdrehsicher angeschraubt werden kann. Auch dieses Zwischenteil 66 besitzt die Riffelung 23.

In Figur 5 ist ein klemmbares Scharniergelenk 67 dargestellt. Dieses Scharniergelenk 67 ist an beliebiger Stelle klemmbar und dient bei Lösung der Klemmung zur Dynamisierung von Gelenken in der Ebene der Scharnierkippbewegung

Der schwenkbare Vierkant 68 ist fest mit dem Schwenkbolzen 69 verbunden, der in einem U-förmig um den Vierkant 68 greifenden Teil 70 gelagert ist. Das über das Teil 70 hinausragende Ende des Schwenkbolzens 69 ist mit einem Gewinde versehen und an zwei sich gegenüberliegenden Seiten flachgefräst. Die auf einer Seite mit einer Radialverzahnung versehene Scheibe 71 ist durch eine entsprechend dem Bolzenquerschnitt ausgebildeten Bohrung verdrehsicher auf diesem Schwenkbolzen 69 aufgebracht.

Eine zwischen dem Teil 70 und der Scheibe 71 um den Schwenkbolzen 69 gewickelte Spiralfeder 73 hält die Scheibe 71 auf Distanz zum Teil 70. Das Teil 70 besitzt an der dieser Scheibe 71 zugewandten Seite ebenfalls eine passende Radialverzahnung. Wird nun die Scheibe 71 mittels der Mutter 72 auf die Verzahnung gepreßt, wird das gesamte Gelenk 67 blockiert. An dem Vierkant 68 und dem Teil 70 sind jeweils eine Aufnahme bzw. ein Stift 16 für die Stiftverbindung vorgesehen, wie sie oben betreffend Figur 2 und Figur 3 beschrieben wurde.

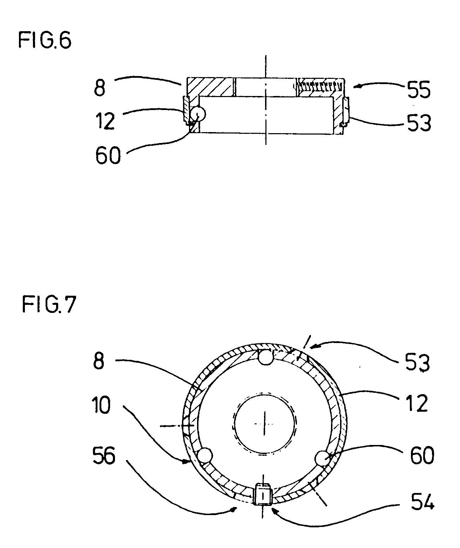

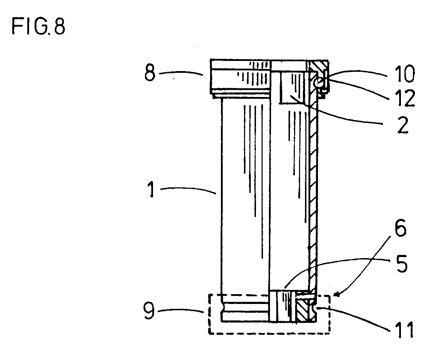

Bei der in Figur 8 dargestellten, teilweise geschnittenen Ansicht des Zentralteiles 1 befindet sich die Verstellmutter 8 bzw. 9 jeweils auf einem Ende des Zentralteils. Die Funktionsweise dieser beider Verstellmuttern 8 und 9 wird nunmehr anhand von Figur 6, 7 und 8 näher beschrieben.

Zur Führung des Vollkolbens, der in Figur 8 nicht dargestellt ist und dem in Figur 1 dargestellten Vollkolben 4 entspricht, dient die entsprechend innenverzahnte Muffe 5, die in das rohrförmige Ende des Zentralteils 1 angepaßt ist. Diese Muffe 5 wird durch eine Madenschraube 6 gesichert, welche in der durchgehenden Innengewindebohrung durch entsprechendes Hineindrehen aus der Innenseite des Innenteilprofils der Muffe 5 heraustritt und somit zusätzlich den Vollkolben 4 klemmen kann.

Die beiden Verstellmuttern 8 und 9 besitzen Innengewinde, die den an den erhabenen Außenteilen der Kolben 3 bzw. 4 vorhandenen Gewindegängen 51 bzw. 52 entsprechen. Diese Vertellmuttern 8 und 9 erlauben eine axiale Verstellung der Kolben 3 und 4 gegenüber dem Zentralteil 1.

Die beiden Verstellmuttern 8 und 9 sind bis auf die gemäß den unterschiedlichen Kolbenquerschnitten ausgebildeten Innengewinde identisch. Die Muttern 8 und 9 umfassen das betreffende Ende des Zentralteils 1 kragenförmig. Dieser Kragen ist an seinem Umfang mit Bohrungen 10 versehen, die der Aufnahme von Kugeln 60 dienen, wobei die sich nach innen verjüngenden Bohrungen 10 den teilweisen Austritt der Kugeln 60 zulassen, jedoch deren Herausfallen verhindern.

Am Umfang beider Zentralteilenden verläuft eine halbrunde Ringnut 11 in Höhe der Kugelmittelebene, so daß die Kugeln 60 darin laufen können. Die Verstellmutter 8 bzw. 9 ist somit mit dem Zentralteil 1 drehbar verbunden. Das Zurückweichen der Kugeln 60 verhindert ein den Kragen (Verstellmutter 8 bzw. 9) umfassender Ring 12, welcher durch einen Sprengring frei drehbar gesichert ist. Durch das Drehen der Verstellmutter 8 bzw. 9 fährt der betreffende Kolben 3 bzw. 4 sinngemäß aus oder ein.

Zum Zwecke der Instrumentierung bzw. der Dynamisierung ist es wünschenswert, die durch die Verstellmutter 8 bzw. 9 gegebene axiale Verbindung zwischen dem Kolben 3 bzw. 4 und dem Zentralteil 1 zu lösen. Hierzu besitzt der Ring 12 gemäß Figur 6 und 7 an seinem Umfang eine der Kugelzahl und -lage entsprechende Anzahl sich nach aussen konisch verengender Bohrungen 53, die - weil in Umfangsrichtung um den gleichen Betrag versetzt - bei Drehen des Ringes 12 mit den Bohrungen 10 im Kragenteil (Verstellmutter 8 bzw. 9) zur Deckung gebracht werden und so mit das Zurückweichen der Kugeln 60 in zentrifugaler Richtung ohne ein Herausfallen erlauben. In Figur 6 und 7 ist die Verstellmutter 8 dargestellt. Die Verstellmutter 9 ist jedoch analog aufgebaut und wirkt mit einem entsprechenden Ring 12 zusammen.

Eine in Höhe der Kugelmittelebene im Kragen befindliche Schraube 54 erlaubt vermittels einer entsprechenden Aussparung 56 am Umfang des Ringes 12 die Wegbegrenzung und Arretierung des Drehringes 12. Eine in der Gewindeebene der Verstellmutter 8 bzw. 9 radialliegende Madenschraube 55 erlaubt die Fixierung der vom Zentralteil 1 gelösten Mutter 8 bzw. 9 an beliebiger Stelle des Kolbens 3 bzw. 4. Hierdurch ist eine begrenzte Dynamisierung der durch den Außenspanner abgestützten Knochenstrecke möglich. Entsprechend der gewählten axialen Wegstrecke werden nur Verformungskräfte von bestimmter Größe auf den Knochen einwirken.

Anstelle dieser eben beschriebenen Verstellmuttern 8 und 9 gemäß Figur 8 sind in Figur 1 die Verstellmuttern 74 und dargestellt, deren Wirkungsweise nunmehr anhand Figur 1 und Figur 9 und 10 näher erläutert werden. In Figur 9 ist die Draufsicht auf das Zentralteil 1 mit eingefahrenem Kolben 3 dargestellt, wobei die Verstellmutter 74 zu sehen ist. Die Wirkungsweise der Verstellmutter 75 ist analog und wird nunmehr nicht mehr im einzelnen beschrieben.

Bei diesen Ausführungsformen können beispielsweise ein oder mehrere Kulissensteine 76 in mindestens einer oder mehreren Nuten des Keilprofils auf dem Kolben 3 laufen. Durch eine kreisringsegmentförmige Vertiefung im Bereich des Gewindes der Verstellmutter 74 wird der Kulissenstein 76 zwangsläufig der axialen Bewegung der Verstellmutter 74 auf dem Kolben 3 folgen. Die auf der Zentralseite herausragende Zunge des Kulissensteines 76 besitzt ebenfalls eine Vertiefung. In diese greift eine in radialer Richtung angebrachte Schraube 77 des Zentralteils 1 so ein, daß sie den Kulissenstein 76 an einer axialen Bewegung hindert.

Ein Verdrehen der Verstellmutter 74 führt somit zu einer sinngemäßen Aus- oder Einwärtsbewegung des betreffenden Kolbens 3. Dreht man die Schraube 77 fest ein, so drückt diese den Kulissenstein 76 fest in die Keilnut, blockiert somit die axiale Bewegungsmöglichkeit des Kolbens 3, bzw. verhindert ein Verdrehen der Verstellmutter 74. Wird die Schraube 77 im Zentralteil 1 herausgedreht, so kann der betreffende Kolben 3 aus dem Zentralteil 1 zum Zwecke der Schnellverstellung herausgezogen werden.

## Patentansprüche

1. Einseitiger verlängerbarer Außenspanner zur Behandlung von Knochenerkrankungen, bei dem aus einem rohrförmigen Zentralteil (1) an beiden Enden mindestens je ein Kolben (3; 4) unabhängig von dem anderen Kolben (4; 3) ausfahrbar angeordnet ist, an dessen äußerem Ende verschiedene Verbindungsteile (59; 62; 66; 67) drehbar befestigbar sind,
dadurch gekennzeichnet,
daß jeder Kolben (3; 4) wahlweise sowohl durch Drehen einer Verstellmutter (8; 9; 74; 75) definiert verstellbar ist als auch teleskopartig verstellbar ist, wobei sowohl bei der definierten als auch bei der teleskopartigen Verstellung die Kolben (3; 4) und das Zentralteil (1) gegen ein Verdrehen gesichert sind.

2. Außenspanner nach Anspruch 1,
dadurch gekennzeichnet,
daß sich am äußeren Ende jedes Kolbens (3; 4) eine zentrale Bohrung (14; 15) befindet, in der der Klemmstift (16) eines der Verbindungsteile (59; 62; 66; 67) befestigbar ist, wobei die Knochenschraubenklemmbacken (61) an dieses Verbindungsteil (59; 62; 66; 67) anschraubbar sind.

3. Außenspanner nach Anspruch 1 oder 2,
dadurch gekennzeichnet,
daß sich zur Verdrehsicherung zwischen dem Zentralteil (1) und dem Verbindungsteil (59; 62; 66; 67) einerseits an der Stirnseite jeden Endes der Kolben (3; 4) eine Verzahnung (20; 21) befindet, andererseits an der korrespondierenden Unterseite des Verbindungsteils (59; 62; 66; 67) eine dazu passende Verzahnung (22; 23) vorhanden ist.

4. Außenspanner nach Anspruch 1, 2 oder 3,
dadurch gekennzeichnet,
daß mindestens einer der Kolben (3) hohlgebohrt ist, so daß der gegenüberliegende Kolben (4) vollständig in diesen eintauchen kann.

5. Außenspanner nach mindestens einem der vorherigen Ansprüche,
dadurch gekennzeichnet,
daß sich im äußeren Teil des Zentralteils (1) und der Kolben (3; 4) mindestens zwei oder mehrere Nuten befinden und daß jeder der ausfahrbaren Kolben eine entsprechende Anzahl in diesen Nuten laufende feste Teile (7) an seinem Umfang besitzt.

6. Außenspanner nach Anspruch 5,
dadurch gekennzeichnet,
daß die sich am Kolbenumfang befindlichen Teile (7) mit Gewindegängen (51, 52) versehen sind und die sich am Zentralteil (1) befindlichen Verstellmuttern (8; 9; 74; 75) in diese Gewindegänge (51, 52) eingreifen und somit die definierte axiale Verstellung der Kolben (3; 4) gegenüber dem Zentralteil (1) bewirken.

7. Außenspanner nach Anspruch 2 bis 6,
dadurch gekennzeichnet,
daß die Knochenschraubenklemmbacken (61) in Längsrichtung oder in Querrichtung anbringbar sind.

8. Außenspanner nach mindestens einem der vorherigen Ansprüche,
dadurch gekennzeichnet,
daß auf dem Zentralteil (1) ein Kreisring (42) lösbar angeordnet ist, der einen Knochensegmentfixateur (63) trägt.

9. Außenspanner nach mindestens einem der vorherigen Ansprüche,
dadurch gekennzeichnet,
daß er ein gelenkiges Verbindungsteil (59) aufweist, das mittels eines Klemmstiftes (16) auf das Ende des Kolbens (3; 4) aufsteckbar ist.

10. Außenspanner nach mindestens einem der vorherigen Ansprüche,
dadurch dekennzeichnet,
daß als Verbindungsteil ein starres Zwischenteil (66) vorgesehen ist, das mittels Klemmstift (16) auf das Ende des Kolbens (3; 4) aufsteckbar ist.

11. Außenspanner nach mindestens einem der vorherigen Ansprüche,
dadurch gekennzeichnet,
daß er als Verbindungsteil ein an beliebiger Stelle klemmbares Scharniergelenk (67) aufweist, das mittels eines Klemmstiftes (16) auf das Ende des Kolbens (3; 4) aufsteckbar ist.

12. Außenspanner nach mindestens einem der vorherigen Ansprüche,
dadurch gekennzeichnet,
daß er als Verbindungsteil einen Umsteller (62) aufweist, der über einen selbsthemmenden Schneckenantrieb die definierte Schwenkbewegung der Plattform (35) ermöglicht und mittels eines Klemmstiftes (16) auf das Ende des Kolbens (3; 4) aufsteckbar ist.

13. Außenspanner nach mindestens einem der vorherigen Ansprüche,
dadurch gekennzeichnet,
daß ein auf dem Zentralteil (1) anflanschbarer Schrittmotor mit Steuereinheit und Stromversorgung über ein Ritzel in die am Umfang der Verstellmutter (8; 9; 74; 75) vorhandene Verzahnung eingreift und diese mit vorgegebener Impulszahl pro Zeiteinheit dreht.

## Claims

1. A unilateral extensible external fixator for the treatment of bone disorders, wherein at least one respective piston (3; 4) is arranged to be extended out of a tubular central portion (1) at each end independently of the respective other piston (4; 3), and in which various connecting portions (59; 62; 66; 67) can be rotatably fixed to the outer end of each piston, characterised in that each piston (3; 4) is selectively definedly displaceable by rotation of an adjusting nut (8; 9; 74; 75) and also telescopically displaceable, wherein the pistons (3; 4) and the central portion (1) are prevented from turning both in the defined displacement and also in the telescopic displacement.

2. An external fixator according to claim 1 characterised in that provided at the outer end of each piston (3; 4) is a central bore (14; 15) in which the clamping peg (16) of one of the connecting portions (59; 62; 66; 67) can be fixed, wherein the bone screw clamping jaws (61) can be screwed to said connecting portion (59; 62; 66; 67).

3. An external fixator according to claim 1 or claim 2 characterised in that for preventing turning as between the central portion (1) and the connecting portion (59; 62; 66; 67) on the one hand a serration (20; 21) is provided at the end face of each end of the pistons (3; 4) and on the other hand a etching serration (22; 23) is disposed at the corresponding underside of the connecting portion (59; 62; 66; 67).

4. An external fixator according to claim 1, claim 2 or claim 3 characterised in that at least one of the pistons (3) is hollow-bored so that the oppositely disposed piston (4) can engage completely into it.

5. An external fixator according to at least one of the preceding claims characterized in that at least two or more grooves are disposed in the outer pert of the central portion (1) and the pistons (3; 4) and that each of the extensible pistons has at its periphery a corresponding number of fixed parts (7) which move in said grooves.

6. An external fixator according to claim 5 characterized in that the parts (7) at the piston periphery are provided with screwthread pitches (51, 52) and the adjusting nuts (8; 9; 74; 75) on the central portion (1) engage into said screwthread pitches and thus produce the defined axial displacement of the pistons (3; 4) relative to the central portion (1).

7. An external fixator according to claims 2 to 6 characterised in that the hone screw clamping jaws (61) can be fitted in the longitudinal direction or in the transverse direction.

8. An external fixator according to at least one of the preceding claims characterised in that a circular ring (42) which carries a hone segment fixator device (63) is releasably arranged on the central portion (1).

9. An external fixator according to at least one of the preceding claims characterised in that it has a pivotable connecting portion (59) which can be fitted by bans of a clamping peg (16) on to the end of the piston (3; 4).

10. An external fixator according to at least one of the preceding claims characterised in that a rigid intermediate portion (66) is provided as a connecting portion, which intermediate portion can be fitted by means of a clamping peg (16) on to the end of the piston (3; 4).

11. An external fixator according to at least one of the preceding claims characterised in that as a connecting portion it has a hinge joint (67) which can be clamped at any location and which can be fitted by means of a clamping peg (16) on to the end of the piston (3; 4).

12. An external fixator according to at least one of the preceding claims characterised in that as a connecting portion it has a transposition device (62) which permits the defined pivotal movement of the platform (35) by way of a self-locking screw drive and which can be fitted by means of a clamping peg (16) on to the end of the piston (3; 4).

13. An external fixator according to at least one of the preceding claims characterised in that a stepping motor which can be flange-mounted on the central portion (1), with control unit and power supply, engages by way of a pinion into the tooth arrangement at the periphery of the adjusting nut (8; 9; 74; 75) and rotates same with a predetermined number of pulses per unit of time.

## Revendications

1. Fixateur externe unilatéral allongeable pour le traitement de maladies osseuses, dans lequel au moins un piston (3 ; 4) est monté, avec une possibilité de sortie indépendamment de l'autre piston (4 ; 3), à chacune des deux extrémités d'une pièce centrale tubulaire (1), diverses pièces de liaison (59 ; 62 ; 66 ; 67) pouvant être fixées en rotation à l'extrémité extérieure dudit piston, caractérisé en ce que chaque piston (3 ; 4) peut être réglé, au choix, aussi bien de manière définie en tournant un écrou de réglage (8 ; 9 ; 74 ; 75) que de manière télescopique, les pistons (3 ; 4) et la pièce centrale (1) étant bloqués en rotation tant avec le réglage défini qu'avec le réglage télescopique.

2. Fixateur externe selon la revendication 1, caractérisé en ce qu'à l'extrémité extérieure de chaque piston (3 ; 4) se trouve un perçage central (14 ; 15), dans lequel peut être fixée la broche de serrage (16) de l'une des pièces de liaison (59 ; 62 ; 66 ; 67), les mâchoires à vis (61) de serrage des os pouvant être vissées sur cette pièce de liaison (59 ; 62 ; 66 ; 67).

3. Fixateur externe selon la revendication 1 ou 2, caractérisé en ce que, pour réaliser un blocage en rotation entre la pièce centrale (1) et la pièce de liaison (59 ; 62 ; 66 ; 67), il est prévu, d'une part, une denture (20 ; 21) sur la face frontale de chaque extrémité des pistons (3 ; 4) et, d'autre part, une denture adaptée (22 ; 23) sur le dessous correspondant de la pièce de liaison (59 ; 62 ; 66 ; 67).

4. Fixateur externe selon la revendication 1, 2 ou 3, caractérisé en ce qu'au moins l'un des pistons, (3), est pourvu d'un perçage, de sorte que le piston opposé (4) peut y pénétrer entièrement.

5. Fixateur externe selon au moins une des revendications précédentes, caractérisé en ce qu'il est prévu au moins deux ou plusieurs gorges dans la partie extérieure de la pièce centrale (1) et des pistons (3 ; 4), et en ce que chacun des pistons prévus pour sortir possède, sur sa périphérie, un nombre correspondant de pièces fixes (7) coulissant dans ces gorges.

6. Fixateur externe selon la revendication 5, caractérisé en ce que les pièces (7) situées à la périphérie des pistons sont munies de pas de vis (51, 52), et les écrous de réglage (8 ; 9 ; 74 ; 75) situés sur la pièce centrale (1) s'engagent dans ces pas de vis (51, 52) et réalisent ainsi le réglage axial défini des pistons (3 ; 4) par rapport à la pièce centrale (1).

7. Fixateur externe selon les revendications 2 à 6, caractérisé en ce que les mâchoires à vis (61) de serrage des os peuvent être montées dans le sens longitudinal ou dans le sens transversal.

8. Fixateur externe selon au moins une des revendications précédentes, caractérisé en ce que, sur la pièce centrale (1), est monté, avec une possibilité de démontage, un anneau circulaire (42) qui porte un fixateur de segment osseux (63).

9. Fixateur externe selon au moins une des revendications précédentes, caractérisé en ce qu'il comporte une pièce de liaison articulée (59) qui peut être emboîtée sur l'extrémité du piston (3 ; 4) au moyen d'une broche de serrage (16).

10. Fixateur externe selon au moins une des revendications précédentes, caractérisé en ce que la pièce de liaison est constituée par une pièce intermédiaire rigide (66) qui peut être emboîtée sur l'extrémité du piston (3 ; 4) au moyen d'une broche de serrage (16).

11. Fixateur externe selon au moins une des revendications précédentes, caractérisé en ce qu'il comporte, à titre de pièce de liaison, une articulation à charnière (67) qui peut être mise en place par serrage en un endroit quelconque et qui peut être emboîtée sur l'extrémité du piston (3 ; 4) au moyen d'une broche de serrage (16).

12. Fixateur externe selon au moins une des revendications précédentes, caractérisé en ce qu'il comporte, à titre de pièce de liaison, un moyen d'ajustement (62) qui, au moyen d'un mécanisme autobloquant à vis sans fin, permet de faire pivoter la plate-forme (35) de manière définie et qui peut être emboîté sur l'extrémité du piston (3 ; 4) au moyen d'une broche de serrage (16).

13. Fixateur externe selon au moins une des revendications précédentes, caractérisé en ce qu'un moteur pas à pas qui est associé à une unité de commande et à un dispositif d'alimentation en courant et qui peut être relié à la pièce centrale (1) s'engage, par l'intermédiaire d'un pignon, dans la denture prévue à la périphérie de l'écrou de réglage (8 ; 9 ; 74 ; 75) et la fait tourner selon un nombre d'impulsions prédéfini par unité de temps.
